# EUROPEAN PATENT APPLICATION

(11) **EP 2 554 198 A1**
(43) Date of publication of application: **06.02.2013**
(21) Application number: 10849073.1
(22) Date of filing: 20.10.2010
(51) Int. Cl.: A61M 5/162, A61M 5/142, A61M 5/168, A61M 39/22

(54) **LIQUID MEDICINE ADMINISTERING DEVICE FOR ENDOSCOPIC SURGERY, MEDICAL CONTROLLER FOR LIQUID MEDICINE, AND LIQUID MEDICINE ADMINISTERING DEVICE FOR ENDOSCOPIC SURGERY COMPRISING SAME**

(30) Priority: 12.07.2010 KR 20100066983; 02.04.2010 KR 20100030201
(71) Applicant: Lee, Sang Yub, Incheon 407-827 (KR)
(72) Inventor: Lee, Sang Yub, Incheon 407-827 (KR)
(74) Representative: Tischner, Oliver
(86) International application number: PCT/KR2010/007172
(87) International publication number: WO 2011/122751

(57) **Abstract**

Disclosed are a medical controller for liquid medicine, and a liquid medicine administering device for endoscopic surgery comprising same. The liquid medicine administering device for endoscopic surgery comprises: a head member including at least one liquid medicine entrance port through which liquid medicine from a syringe is admitted, and a first gas entrance port into which gas enters from a gas supply line and which is connected to the liquid medicine entrance port; a strong member connected to the head member, and including a liquid medicine passage inserted into a human body for discharging the liquid medicine to a surgical site; and a valve unit installed on the head member to selectively communicate the liquid medicine passage with the liquid medicine entrance port or the first gas entrance port. The liquid medicine that enters through the liquid medicine entrance port is through the liquid medicine passage by means of air provided from the first as entrance port and is discharged to the surgical site.

## Description

### CROSS-REFERENCES TO RELATED APPLICATION

This patent application is a U.S. national phase under 35 U.S.C 371 of PCT/KR2010/007172 filed on October 20, 2010, which claims the benefit of priority from Korean Patent Applications Nos. 10-2010-0030201 and 10-2010-0066983, filed on April 2, 2010, and July 12, 2010, and the contents of which are incorporated herein by reference in its entirety.

### BACKGROUND OF THE INVENTION

### 1. Field of the Invention

The present invention relates to a liquid medicine controller for controlling the injection or administration of liquid medicine for medical use, and a liquid medicine administering device for endoscopic surgery comprising the same.

### 2. Description of the Related Art

The endoscopic surgery, unlike conventional laparotomy, is practiced by inserting an endoscopic apparatus having 2 meter or so of length into a human body through oral or rectal cavity, with monitoring a surgical site through various endoscopic tools. When bleeding occurs at incision site during endoscopic surgery, a practitioner seals off the incision using high voltage of electrocautery. Because the electric voltage is used to seal off the surgical site, a patient can have severe pains.

Accordingly, study has been conducted to find a way of applying liquid medicine on a bleeding site to stop the bleeding and seal off the site during endoscopic surgery. Korean Pat No. 10-2010-0018377 discloses an apparatus for injecting liquid medicine for laparoscopic surgery, which injects liquid medicine to stop bleeding or seal off a surgical site during laparoscopic surgery. According to this liquid medicine injecting apparatus, liquid medicine filled in a syringe is flowed through an inflow port into an inner tube of a tubular member and discharged to the surgical site through a discharge port formed at an end of the inner tube.

In such a conventional liquid medicine injecting apparatus, the liquid medicine is fully filled in the inner tube by the syringe to be discharged through the discharge port at the end of the inner tube. Accordingly, a considerable amount of liquid medicine may remain unused for the surgical site after the procedure, and the liquid medicine remaining in the inner tube is altered over time and therefore, cannot be used for the next operation.

Meanwhile, in the case of laparoscopic surgery, the tool for applying liquid medicine is 20cm ∼ 30cm in length. However, since the tool for use in gastroscopic or colonscopic surgery is at least 180cm in length, liquid medicine is hardly fully chargeable up to the end of the endoscopic tool. Even when a large amount of liquid medicine is used for the procedure, the amount remaining in the tool is still larger than the liquid medicine that is actually used. In other words, the conventional liquid medicine administering apparatus has shortcoming of unnecessary waste of medicine, because it is difficult to use liquid medicine in an amount that is actually used for the surgical site. Furthermore, considering relatively high price of the liquid medicine to stop bleeding and prevent adhesion, cost for surgery increases because cost for the liquid medicine is unnecessarily incurred.

### DETAILED DESCRIPTION OF THE INVENTION

### Technical object

The invention has been made to overcome the above-mentioned problems occurring in the prior art, and accordingly, it is an object of the present invention to provide an apparatus for introducing liquid medicine for endoscopic surgery, which uses an exact amount of liquid medicine necessary for a wound, without causing unnecessary waste of liquid medicine.

Further, an object of the present invention is to provide an apparatus for introducing liquid medicine, which is safely and easily inserted into a human body.

Further, an object of the present invention is to provide a liquid medicine controller which controls introducing of at least two liquid medicines, and also selectively controls introducing of air.

Further, an object of the present invention is to provide an apparatus for introducing liquid medicine with improved structure, to which a liquid medicine controller can be selectively connected and used.

### Means to solve the object

In order to achieve the above-mentioned object, a liquid medicine administering apparatus for endoscopic surgery in one embodiment may include a head member comprising one or more liquid medicine inflow ports through which liquid medicine is introduced from a syringe, and one or more first gas inflow ports which are connected to the liquid medicine inflow ports and through which a gas from a gas supply line is introduced, a tubular member connected to the head member and inserted into a human body, the tubular member comprising a liquid medicine passage through which the liquid medicine is discharged onto a surgical site, and a valve unit installed on the head member to selectively connect the liquid medicine passage to the liquid medicine inflow ports or the first gas inflow ports. The liquid medicine introduced through the liquid medicine inflow ports is moved along the liquid medicine passage by an air provided from the gas inflow ports, to be discharged onto the surgical site.

The tubular member may include an inner tube forming the liquid medicine passage, an outer tube having a passage to receive the inner tube therein. The head member is slidably provided on the outer tube to cause an end of the inner tube to be selectively protruded outside an end of the outer tube.

The one or more liquid medicine inflow ports may include a first liquid medicine inflow port connected to a first syringe filled with a first liquid medicine, and a second liquid medicine inflow port connected to a second syringe filled with a second liquid medicine, and the one of more first gas inflow ports may include a first gas inflow pipe connected to the first inflow port, and a second gas inflow pipe connected to the second inflow port.

The liquid medicine passage formed in the inner tube is partitioned into a first liquid medicine passage and a second liquid medicine passage through which the first and second liquid medicines are flowed, respectively. The first liquid medicine passage is connected to the first liquid medicine inflow port and the first gas inflow pipe, and the second liquid medicine passage is connected to the second liquid medicine inflow port and the second gas inflow pipe. The valve unit may include a first valve body which selectively connects the first liquid medicine passage to the first liquid medicine inflow port or to the first gas inflow pipe, a second valve body which selectively connects the second liquid medicine passage to the second liquid medicine inflow port or to the second gas inflow pipe, and an operating lever which connects the first and second valve bodies for concurrent operating of the first and second valve bodies.

The first and second liquid medicines may include a skin adhesive component.

The head member may additionally include a second gas inflow port connected to the gas supply line, and the tubular member has a passage formed between the inner tube and the outer tube to allow the air provided from the second gas inflow port to be discharged along with the liquid medicine at an end where the air and the liquid medicine are discharged.

In one embodiment, a liquid medicine controller may include at least two liquid medicine inlets, at least two liquid medicine outlets connected to the liquid medicine inlets, and a valve member which connects the at least two liquid medicine inlets and the at least two liquid medicine outlets, or connects the at least two air inlets and the at least two liquid medicine outlets.

The valve member connects the liquid medicine inlets to the liquid medicine outlets and at the same time, blocks the air inlets from the liquid medicine outlets, or connects the air inlets to the liquid medicine outlets and at the same time, blocks the liquid medicine inlets from the liquid medicine outlets.

The valve member is rotatably provided between the liquid medicine inlets and the liquid medicine discharge ports.

The valve member may include a tubular valve body and a lever connected to the tubular valve body to be manually operated by a user, and the tubular valve body comprises a plurality of passing holes formed therein.

In one embodiment, a liquid medicine administering apparatus for endoscopic surgery is provided, which may include a syringe unit, a liquid medicine controller, and a catheter unit connected to the liquid medicine controller. The liquid medicine controller may include at least two liquid medicine inlets, at least two liquid medicine outlets connected to the liquid medicine inlets, and a valve member which connects the at least two liquid medicine inlets and the at least two liquid medicine outlets, or connects the at least two air inlets and the at least two liquid medicine outlets.

The catheter unit may include a connecting member separably connected to the liquid medicine controller, an inner casing connected to the connecting member, a first and a second tubes provided in an interior space of the inner casing, and an outer casing formed to surround at least part of the inner casing.

The outer casing is slidable in a lengthwise direction with respect to the inner casing.

The liquid medicine administering apparatus may additionally include a third tube connected to the outer casing to surround the first and second tubes therein.

### Effect of the invention

According to the above embodiments, because a proper amount of liquid medicine suitable for use on a wound is inserted and delivered by a gas such as air to be discharged onto the wound, the liquid medicine administering apparatus provides advantages of less waste of liquid medicine and reduced cost.

Further, because an end of an inner tube is selectively exposed to outside an end of an outer tube, a tubular member of the liquid medicine administering apparatus according to the embodiments can be stably and easily inserted into a patient's body.

According to the embodiments, the liquid medicine controller provides convenience of use, because the liquid medicine controller is attachable to and removable from a catheter unit, and can concurrently control two liquid medicines, while this also controls so that air and liquid medicine are selectively introduced.

According to the embodiments, because liquid medicine is carried by air to the wound site and discharged, unnecessary waste of liquid medicine is reduced, and cost for the use of liquid medicine also decreases.

According to the embodiments, due to structure in which an outer casing is flexibly provided with respect to an inner casing, it is convenient to adjust the length of the catheter unit and convenience of use improves.

### BRIEF DESCRIPTION OF THE DRAWINGS

The above and/or other aspects and advantages of the present invention will become apparent and more readily appreciated from the following detailed description, taken in conjunction with the accompanying drawings of which:
FIG. 1 is a perspective view of a liquid medicine administering apparatus according to an embodiment of the present invention;
FIG. 2 illustrates the liquid medicine administering apparatus from a direction A indicated in FIG. 1;
FIG. 3 is a cross-section view of the liquid medicine administering apparatus of FIG. 1;
FIG. 4 is a partial side cross-section view illustrating a first and second liquid medicine inflow ports and first and second channels being connected to each other by a valve unit of FIG. 1;
FIG. 5 is a partial cross-section view illustrating first and second gas inflow tubes and first and second channels being connected to each other by the valve unit of FIG. 1;
FIG. 6 illustrates a situation in which an end of an inner tube being placed within an outer tube of FIG. 1;
FIG. 7 illustrates a situation in which an end of the inner tube being exposed outside the end of the outer tube of FIG. 1;
FIG. 8 is a perspective view of a liquid medicine administering apparatus according to an embodiment of the present invention;
FIGS. 9A and 9B are side views of the liquid medicine administering apparatus of FIG. 8, in which FIG. 9A is a partially-enlarged cross-section of a first control unit 450 in a state that a lever of the liquid medicine controller is lifted, and an outer casing of a catheter unit is lifted above an inner casing, and FIG. 9B is a partially-enlarged cross-section of the first control unit 450 in a state that the lever of liquid medicine controller is lowered and the outer casing of the catheter unit is slid with respect to the inner casing down to a lower portion;
FIG. 10 is an exploded perspective view of an liquid medicine controller according to an embodiment of the present invention;
FIG. 11 is an exploded view of the catheter unit of FIG. 8; and
FIG. 12 is a cross-sectional view of the catheter unit of FIG. 8.

### BEST MODE

Reference will now be made in detail to the embodiments of the present invention, examples of which are illustrated in the accompanying drawings, wherein like reference numerals refer to the like elements throughout. The embodiments are described below to explain the present invention by referring to the figures.

The matters defined in the description, such as detailed construction and elements, are provided to assist in a comprehensive understanding of the invention. Thus, it is apparent that the exemplary embodiments of the present invention can be carried out without those specifically defined matters. Also, well-known functions or constructions are not described in detail since they would obscure the invention with unnecessary detail.

An apparatus 10 for introducing liquid medicine for endoscopic surgery according to an embodiment will be explained below with reference to accompanying drawings.

Modifications of the embodiment are possible, and the scope of the invention is not limited to the specific embodiments explained below. The embodiments are provided to those with average knowledge in the pertinent area to more fully explain the present invention. Accordingly, the configurations of the constituents in the drawings may be exaggerated for the purpose of convenience of explanation.

As used herein, the 'apparatus for introducing liquid medicine for endoscopic surgery' may refer to an apparatus to be inserted into oral or rectal cavity of a patient to apply liquid medicine to a surgical site during endoscopic surgery, because it is impossible to spray the liquid medicine to stop bleeding or seal off incision site directly from a syringe.

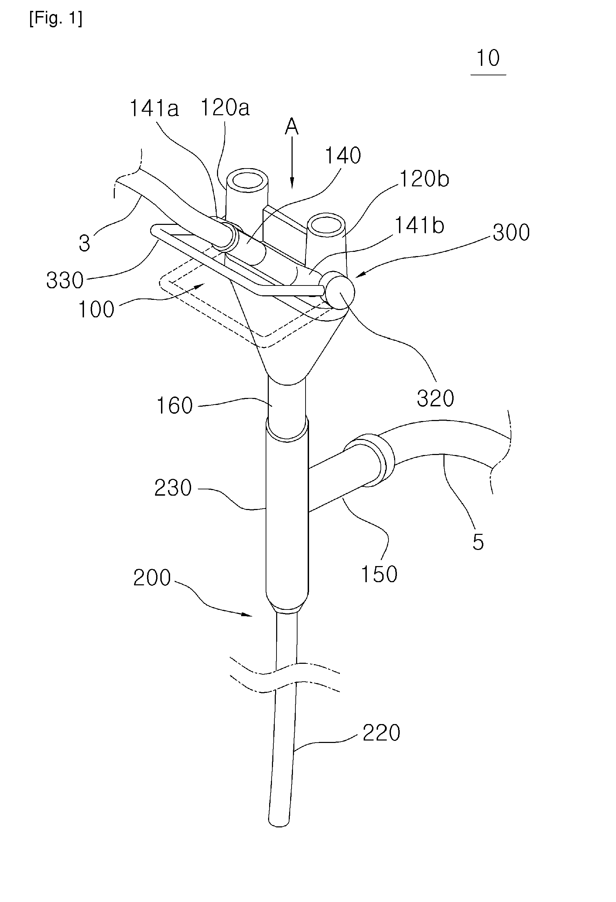

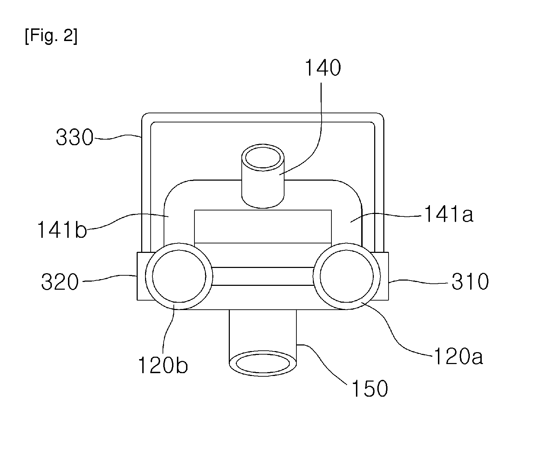

Referring to FIGS. 1 to 3, the apparatus 10 for introducing liquid medicine for endoscopic surgery according to an embodiment may include a head member 100, a tubular member 200 and a valve member 300.

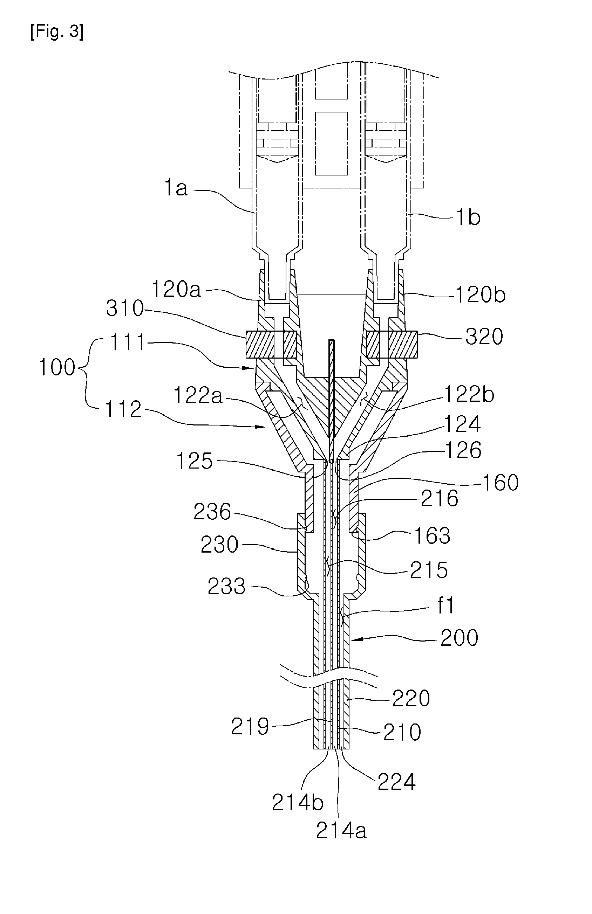

The head member 100 may include a first body 111 and a second body 112. The first body 111 may include a first liquid medicine inflow port 120a connected to a first syringe 1a (FIG. 3), and a second liquid medicine inflow port 120b connected to a second syringe 1b (FIG. 3), and a liquid medicine outflow port 124 connected to an inner tube 210 of the tubular member 200. The liquid medicine outflow port 124 may include a first hole 125 connected to the first liquid medicine inflow port 120a via a first channel 122a, and a second hole 126 connected to the second liquid medicine inflow port 120b via a second channel 122b. The first hole 125 is connected to a first liquid medicine passage 215 of the inner tube 210, and the second hole 126 is connected to a second liquid medicine passage 216 of the inner tube 210.

Further, the first body 111 includes a first liquid medicine delivering gas tube 141a connected to the first liquid medicine inflow port 120a, a second liquid medicine delivering gas tube 141b connected to the second liquid medicine inflow port 120b, and a first gas inflow port 140 to provide gas to the first and second liquid medicine delivering gas tubes 141a, 141b. The first gas inflow port 140 is connected to a gas supply line 3 (see FIG. 1).

The second body 112 is formed into a flat funnel shape, and covers all the remaining portions other than the first and second liquid medicine inflow ports 120a, 120b. A connecting port 160, connected to the tubular member 200, is formed under the second body 112.

The tubular member 200 is to be inserted into a patient's body through oral or rectal cavity and discharge liquid medicine onto a surgical site, and has approximately 2 meters of length. ]The tubular member 200 includes an inner tube 210, an outer tube 220 and a cap member 230.

The inner tube 210 is connected to the liquid medicine outflow port 124 of the head member 100 in a state of being inserted into the outer tube 220.

The inner tube 210 includes a partition 219 to define two spaces. That is, the inner tube 210 is divided into the first liquid medicine passage 215 and the second liquid medicine passage 216 by the presence of the partition 219, and a first and a second discharge ports 214a, 214b, through which liquid medicine is discharged, are formed on an end of the inner tube 210.

The outer tube 220 is connected to the connecting port 160 formed under the second body 112 of the head member 100. The outer tube 220 plays a role of protecting the inner tube 210, and also a role of supply line by guiding the gas from the second gas inflow port 150 to the location of discharging the liquid medicine. The outer tube 220 has a passage which is sized to have a larger inner diameter than an outer diameter of the inner tube 210.

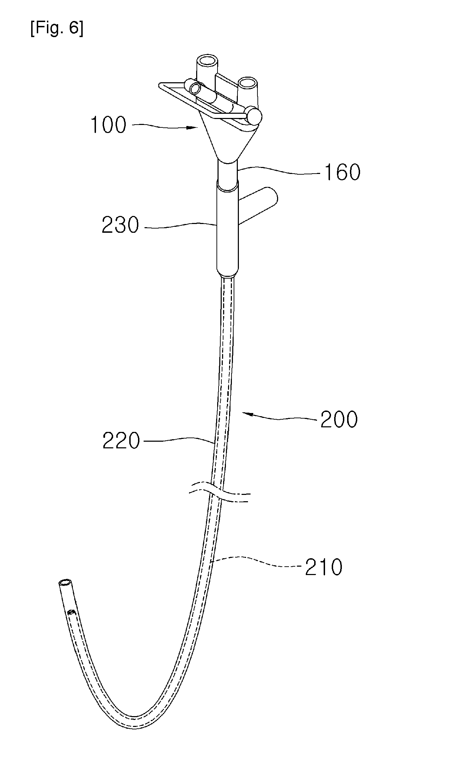

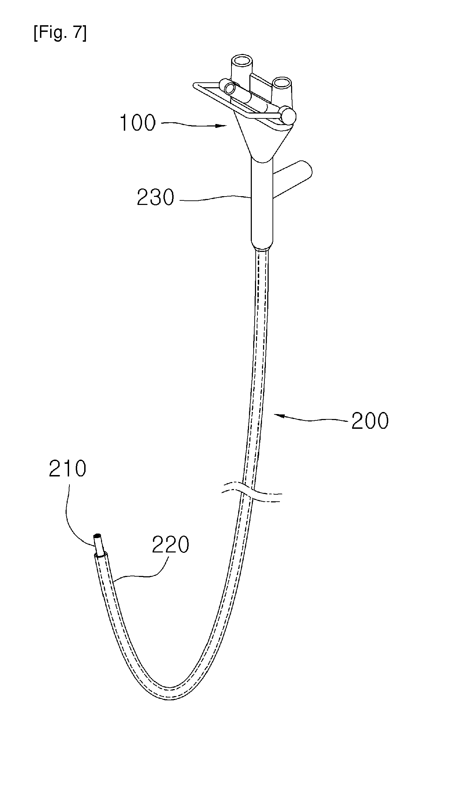

The cap member 230 has a larger diameter than the outer tube 220 and is integrally formed on one end of the outer tube 220. A first and a second protrusions 233, 236 are formed at predetermined distance from each other on an inner surface of the cap member 230. A connecting port 160 is slidably inserted on the inner surface of the cap member 230. In this example, a groove 163 is formed in an end of the connecting port 160 to be snapped with one of the first and second protrusions 233, 236 so that the connecting port 160 is in half-fixed state. That is, the half-fixed state is made when a practitioner grabs the cap member 230 and pushes the head member 100, causing the connecting port 160 to move so that the groove 163 is snapped with the first protrusion 233, in which state the end of the inner tube 210 is protruded to be exposed outside the end of the outer tube 220 (see FIG. 7). On the contrary, if the practitioner grabs the cap member 230 and pulls the head member 100, the groove 163 of the connecting port 160 is separated from the protrusion 230 and moved to be snapped with the second protrusion 236. Accordingly, the end of the inner tube 210 is placed within the outer tube 220 as illustrated in FIG. 6.

The second gas inflow port 150, connected to the gas supply line 5 (FIG. 1), is formed on an outer surface of the cap member 230. The air provided from the second gas inflow port 150 flows into the cap member 230 and advances through a passage f1 formed between the inner surface of the outer tube 220 and the outer surface of the inner tube 210 to be discharged at the discharge port 224 on the end of the outer tube 220 along with the liquid medicine.

Referring to FIGS. 1, 3 to 5, the valve unit 300 includes a first valve body 310, a second valve body 320 and an operating lever 330. The first valve body 310 is provided to selectively connect the first liquid medicine passage 215 to either the first liquid medicine inflow port 120a or the first liquid medicine delivering gas tube 141a, and the second valve body 320 is provided to selectively connect the second liquid medicine passage 216 either to the second liquid medicine inflow port 120b or the second liquid medicine delivering gas tube 141b.

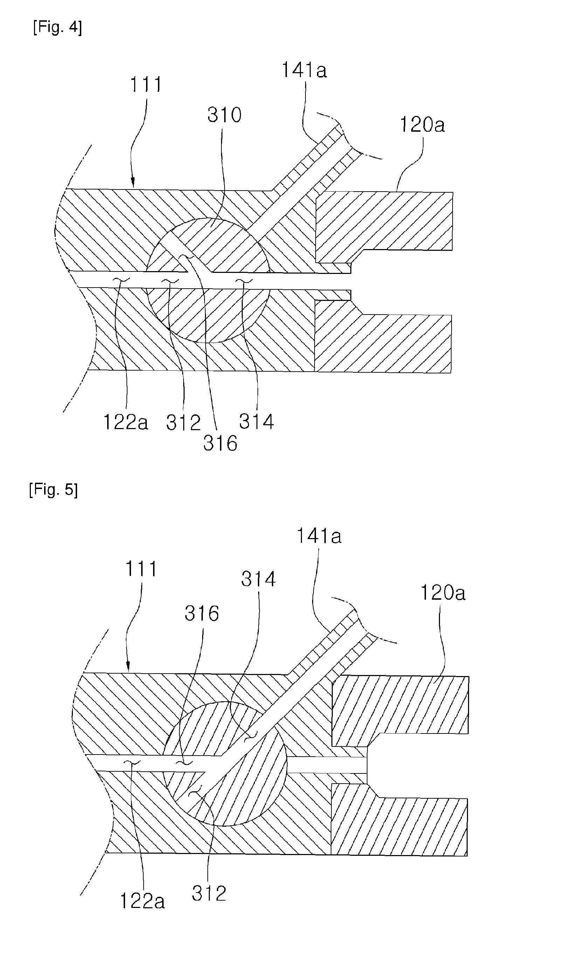

The first and second valve bodies 310, 320 have identical structures, and accordingly, only the first valve body 310 will be explained below for the sake of brevity. Referring to FIG. 4, the first valve body 310 is provided in a cylindrical configuration, and is rotatable within the first body 111. The first valve body 310 includes a first connecting passage 312, a second connecting passage 314 and a third connecting passage 316 which are pierced from an outer circumference to the center at predetermined angle to each other.

The operating lever 330 is formed into "⊏" shape, in which both ends are connected to both sides of the first valve body 310 and the second valve body 320 to rotate the first and second valve bodies 310, 320. Accordingly, the first and second valve bodies 310, 320 are operated concurrently in accordance with manipulation on the operating lever 330.

When the operating lever 330 is almost at right angle to the first body 111, referring to FIG. 4, the first channel 122a and the first liquid medicine inflow port 120a are connected through the first connecting channel 312 and the second connecting channel 314, while as the operating lever 330 is turned to a predetermined angle as illustrated in dotted line in FIG. 1, the first and second valve bodies 310, 320 are turned concurrently so that, as illustrated in FIG. 5, the first liquid medicine delivering gas tube 141a and the first channel 122a are connected by the second connecting channel 314 and the third connecting channel 316.

In one embodiment, the first and second liquid medicines respectively charged in a first syringe 1a and a second syringe 1b, are supplied through independent routes from each other to prevent mixture thereof before the discharge onto the surgical site. The first and second liquid medicines are adhesive components which are mixed with each other upon being discharged onto the surgical site, causing coagulation within a few seconds. By way of example, the first and second liquid medicines may be coagulating components such as fibrinogen and thrombin for use in stopping bleeding at a surgical site and sealing off a wound.

The operation of the liquid medicine administering apparatus 10 according to an embodiment of the present invention will be explained below with reference to FIGS. 1 to 7.

The first and second syringes 1a, 1b are connected to the first and second liquid medicine inflow ports 120a, 120b of the liquid medicine administering apparatus 10, and the gas supply lines 3, 5 are connected to the first and second gas inflow ports 140, 150, respectively. The tubular member 200 is inserted into a patient's body, and so positioned that the end of the tubular member 200 is located close to the surgical site.

Referring to FIG. 6, in inserting the tubular member 200, the practitioner may grab the cap member 230 and pull the head member 100 to place the inner tube 210 within the outer tube 220, in which case the patient's body or a channel for inserting endoscopic tool is prevented from being damaged by the inner tube 210. After that, in applying liquid medicine with the tubular member 200 being placed close to the wound, referring to FIG. 7, the practitioner grabs the cap member 230 and pushes the head member 100 to thus cause the end of the inner tube 210 to protrude outside the end of the outer tube 200.

When prepared for the procedure, the practitioner presses pistons of the first and second syringes 1a, 1b, which are charged with the first and second liquid medicines, so that the liquid medicines are supplied to the liquid medicine administering apparatus 10 in the amounts as needed for the surgical site.

The first and second liquid medicines are partially filled in the first and second liquid medicine passages 215, 216. That is, the first and second liquid medicines are charged onto to a predetermined upper portion of each of the first and second liquid medicine passages 215, 216. As the practitioner operates the operating lever 330 to turn the first and second valve bodies 310, 320, the channel is altered so that the first and second liquid medicine passages 215, 216 are connected to the first and second liquid medicine delivering gas pipes 141a, 141b. At this time, by pedal-operating a switch (not illustrated) of the gas supplying line 3 to supply gas, the gas is discharged through the discharge port 224 and thus is sprayed onto the surgical site along with the first and second liquid medicines coming out of the first and second discharge ports 214a, 214b of the inner pipe 210.

As explained above, the liquid medicine administering apparatus 10 according to an embodiment is used in a manner in which an exact amount of liquid medicine that is necessary for a wound area is introduced with the syringes 1a, 1b, the first liquid medicine delivering gas tube 141a and the first liquid medicine passage 215 are connected to each other, and the second liquid medicine delivering gas tube 141b and the second liquid medicine passage 216 are connected to each other according to manipulation on the valve unit 300, and the exact amount of liquid medicine necessary for the surgical site, which is provided from the syringes 1a, 1b, is delivered through the first and second liquid medicine passages 215, 216 and sprayed onto the wound area through the first and second discharge ports 214a, 214b by the introduction of gas through the first gas inflow port 140.

However, the conventional liquid medicine administering apparatus has shortcomings in which, because the liquid medicines from the syringes have to be fully charged into the inner tube until the liquid medicine is discharged through the discharge port of the inner tube according to pushing on the pistons of the syringes, the amount of liquid medicine supplied from the syringe exceeds the amount that is actually used on the wound site, thereby leaving un-used liquid medicine in the inner tube, which degrades over time to unusable state. The present invention overcomes the shortcomings of the conventional art, because exact amount of liquid medicine for use on the wound site is supplied from the syringes 1a, 1b through the first and second liquid medicine passages 215, 216, and then further delivered to the discharge ports 214a, 214b by being pushed by the gas.

Further, because the liquid medicine administering apparatus 10 for endoscopic surgery according to an embodiment has a structure in which the connecting port 160 of the head member 100 is slidably moveable between the outer tube 220 and the cap member 230 so that the inner tube 210 is selectively exposed to the outer tube 220, the end of the inner tube 210 is not exposed outside through the end of the outer tube 220 when the tubular member 200 is inserted into a patient's body, thereby preventing the end of the inner tube 210 from damaging the patient's body or channel for inserting endoscopic tool.

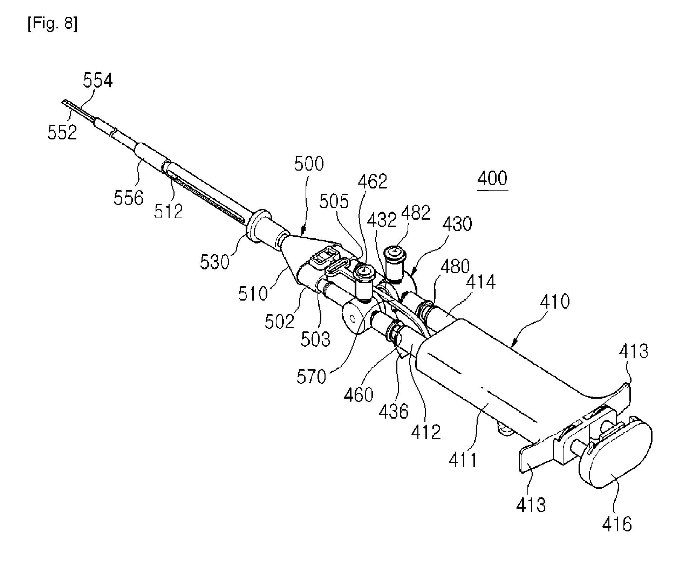

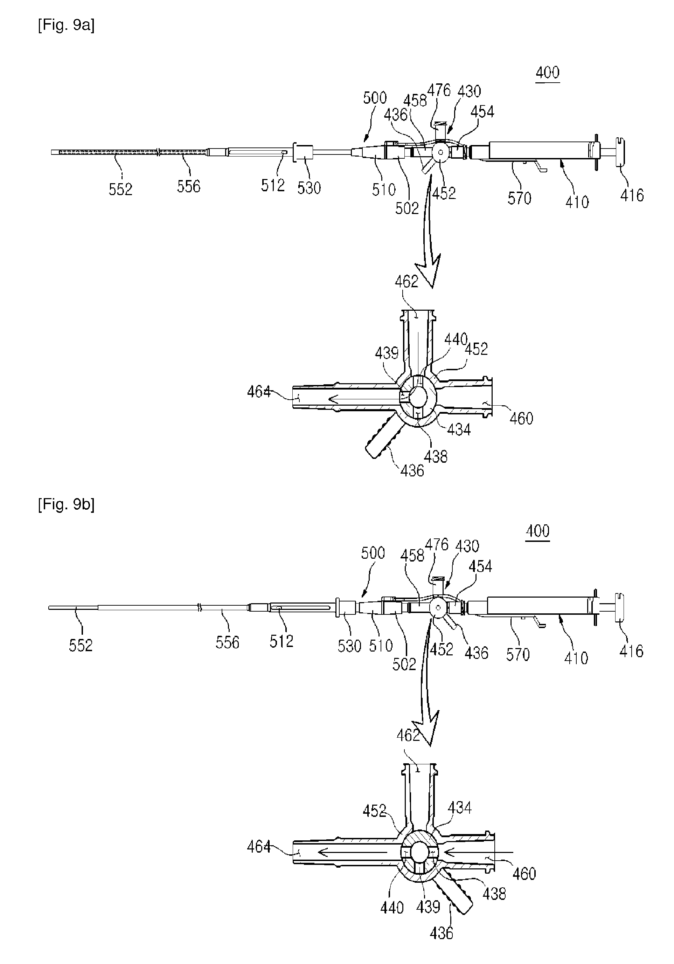

Referring to FIGS. 8, 9A and 9B, the liquid medicine administering apparatus 400 according to an embodiment may include a syringe unit 410, a liquid medicine controller 430 and a catheter unit 500.

The syringe unit 410 includes a first and a second syringes 412, 414, a knob 416, and a supporting member 411 which connects the first and second syringes 412, 414. The first and second syringes 412, 414 are filled with liquid medicine, and one ends of the first and second syringes 412, 414 are inserted into a first and a second liquid medicine inlets 460, 480, respectively. The knob 416 is separably connected to the other ends of the first and second syringes 412, 414. The user pushes the knob 416 with his thumb in order to introduce the liquid medicine. The supporting member 411 includes a fixing groove (not illustrated) to separably fix the firs and second syringes 412, 414, so that the respective syringes 412, 414 are connected to and separated from the fixing groove (not illustrated). A protrusion 413 extends in left and right directions on an upper end of the supporting member 411, and plays a role of supporting user's rest fingers when the user pushes the knob 416 with his or her thumb.

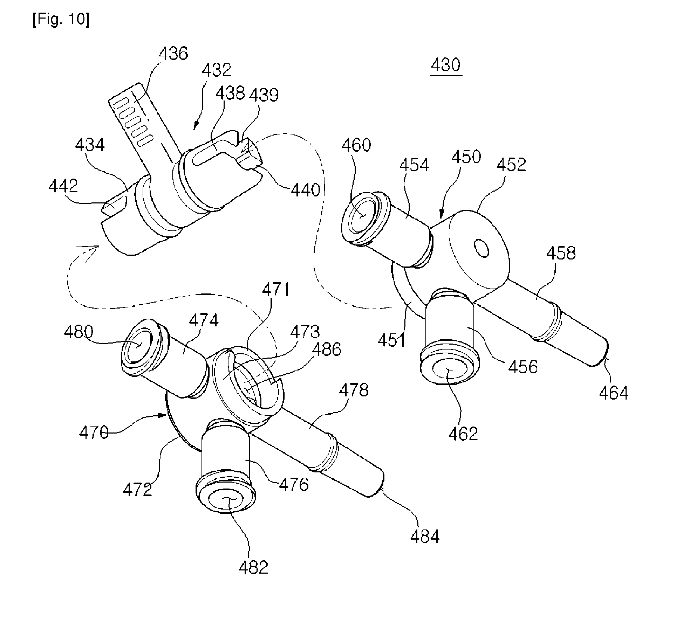

The liquid medicine controller 430 is provided to concurrently control two types of liquid medicines introduced from the syringe unit 410,and referring to FIG. 10, includes a valve member 432, and a first and a second control units 450, 470.

The valve member 432 includes a valve body 434 and a lever 436. The left and right halves of the valve body 434 are in symmetrical relation with each other with reference to the lever 436. The valve body 434 includes three passing holes 438, 439, 440, 442 formed at an angle of 90 degrees from one another other on each side thereof. Although there are first to fourth passing holes 438, 439, 440, 442 illustrated in FIG. 10, the fifth and sixth passing holes (not illustrated) are also formed at a location of the second and a symmetrically corresponding location of the third. The passing holes may each be formed as an elongated hole with one open end, but may be shaped otherwise, such as circular or polygonal hole. The lever 436 protrudes from the center of the valve body 343 to a predetermined direction to outside, so that the user is able to grab the valve body 434 when seating the same on a valve seating portion 486.

Referring to FIG. 10, the first and second control units 450, 470 includes a first and a second bodies 452, 472, a first and a second inflow pipes 454, 474, a first and a second air pipes 456, 476, and a first and a second discharge pipes 458, 478.

The first and second bodies 452, 472 are formed symmetrically to each other, and have the valve seating portion 486 provided therein. The first and second bodies 452, 472 are in cylindrical form with one open side. A second connecting portion 473 and a second cutaway portion 471 are formed on one open side of the second body 472. Upon connecting to the first body 452, the second connecting portion 473 is inserted inward the first connecting portion 451, and the second cutaway portion 471 is contacted with the first cutaway portion (not illustrated) to define a groove through which the lever 436 of the valve member 432 is moved.

The first and second inflow pipes 454, 474 include the first and second liquid medicine inlets 460, 480 formed on one ends thereof. The other ends of the first and second inflow pipes 454, 474 are connected to the first and second bodies 452, 472. When the liquid medicine is introduced into the first and second liquid medicine inlets 460, 480, the liquid medicine is drawn into the first and second bodies 452, 472 by the pressure and weight.

The first and second air pipes 456, 476 are at an angle of 90 degrees with the first and second introducing pipes 454, 474, with one ends being connected to the first and second bodies 452, 472 and the other ends having a first and a second air inlets 462, 482 formed therein. Although not illustrated, the first and second air inlets 462, 482 are connected to an air supply pipe (not illustrated) to supply air.

The first and second discharge pipes 458, 478 are at symmetrical locations to the first and second inflow pipes 454, 474 with reference to the first and second bodies 452, 472. A first and a second discharge ports 464, 484 are formed on one ends of the first and second discharge pipes 458, 478, respectively.

Referring to FIGS. 8 to 9B, 11 and 12, the catheter unit 500 includes a connecting member 502, an inner casing 510, an outer casing 530, and a first to third tubes 552, 554, 556.

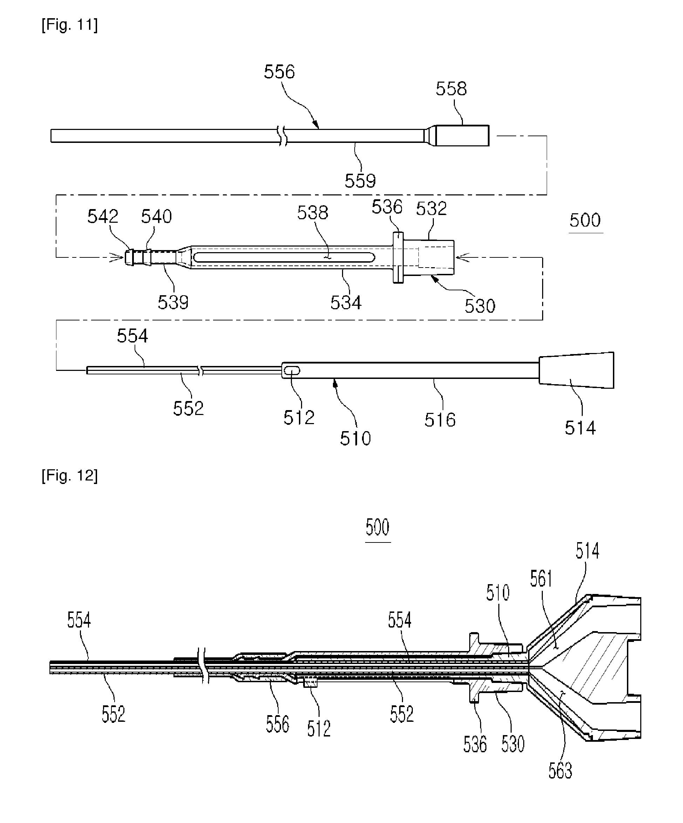

The connecting member 502 includes a first and a second entrances 503, 505 for fluid communication with the first and second liquid medicine discharge ports 464, 484 (see FIG. 12), and ends of the first and second discharge pipes 458, 478 are separably inserted into the first and second entrances 503, 505. The connecting member 502 is fixedly connected to the inner casing 510. As the The connecting member 502 is inserted into and fixed within the head portion 514 of the inner casing 510, channels 561, 563 are formed for the liquid medicine to flow therethrough.

Referring to FIGS. 11 and 12, the inner casing 510 includes the head portion 514, a body portion 516, and a first and a second tubes 552, 554. The head portion 514 is shaped in triangular form, in which one end is connected to the connecting member 502 and extended. The head portion 514 is integrally formed with the body portion 516. The body portion 516 is integrally connected to the other end of the head portion 514, and the first and second tubes 552, 554 are formed in a lengthwise direction on an inner surface of the body portion 516 and elongated outside the other ends of the body portion 516. The first and second tubes 552, 554 are passed through the interior of the inner casing 510, the outer casing 530 and the third tube 556.

The outer casing 530 is so formed to surround the inner casing 510, and slidable with respect to the inner casing 510. The outer casing 530 includes the head portion 532, a handle portion 536 and a body portion 534. The head portion 532, the handle portion 536 and the body portion 534 are integrally formed with each other, and the body portion 532 includes a long hole 538 extended in a lengthwise direction. Upon connecting with the inner casing 510, the stopper 512 of the body portion 516 of the inner casing 510 protrudes through the long hole 538, and the length of the long hole 538 is within a range of sliding movement of the outer casing 530. The body portion 534 has a narrowing tubular portion 539 with gradually decreasing outer diameter formed on a lower portion thereof, and the narrowing tubular portion 539 includes a first and a second locking jaws 540, 542 formed thereon.

The third tube 556 includes a head portion 558 and a body portion 559, and the head portion 558 is connected to the narrowing tubular portion 539 of the outer casing 530. The head portion 558 is connected with the narrowing tubular portion 539 and fixed in the first and second locking jaws 540, 542.

Referring to FIGS. 8 to 12, the operation of the liquid medicine controller 430 and the liquid medicine administering apparatus 400 having the liquid medicine controller 430 according to the present invention will be explained below in detail.

In order to inject liquid medicine into a patient's body during surgery, a practitioner charges liquid medicine in the first and second syringes 412, 414, respectively, and places the lever 436 of the liquid medicine controller 430 at a position shown in FIG. 9A. When the lever is in the position of FIG. 9A, the first and second liquid medicine inlets 460, 480 and the first and second liquid medicine discharge ports 464, 484 are connected to each other by the valve member 432, while the first and second air inlets 462, 482 and the first and second liquid medicine discharge ports 464, 484 are blocked from each other (see partially-enlarged cross-section of FIG. 9A). In this state, as the user presses the knob 416, the liquid medicine is introduced into the first and second liquid medicine inlets 460, 480 of the liquid medicine controller 430. Accordingly, the liquid medicine is introduced into the first control unit 450, passed through the first and third passing holes 438, 440 of the valve member 432, and discharged through the first liquid medicine discharge port 464 of the first discharge pipe 458. The liquid medicine introduced into the second control unit 470 is passed through the fourth passing hole 442 and the seventh passing hole (not illustrated) at a symmetric location of the valve member 432, and discharged through the second liquid medicine discharge port 484 of the second discharge pipe 478. The liquid medicines discharged through the first and second liquid medicine discharge ports 464, 484 are passed through the first and second channels 561, 563 and introduced into the patient's body through the first and second tubes 552, 554 (see FIG. 12).

If the user finishes using the liquid medicine administering apparatus 400 or wishes to change the liquid medicine, it is necessary to discharge the liquid medicine remaining in the liquid medicine administering apparatus 400. To discharge the remaining liquid medicine from the liquid medicine administering apparatus 400, the user moves the lever 436 in a downward direction as illustrated in FIG. 9B. With the lever in the position as illustrated, the first and second liquid medicine inlets 460, 480 and the first and second liquid medicine discharge ports 464, 484 are blocked from each other, while the first and second air inlets 462, 482 and the first and second liquid medicine discharge ports 464, 484 are connected to each other (see partially-enlarged cross-section of FIG. 9B). Accordingly, the air is introduced into the first and second air inlets 464, 482, introduced into the first and second channels 561, 563 of the catheter unit 500 through the first and second liquid medicine discharge ports 464, 484 (see FIG. 12), thereby pushing out the remaining liquid medicine from the interior of the liquid medicine controller 430 and the catheter unit 500 outside the patient's body through the first and second tubes 552, 554.

To introduce liquid medicine, the user raises the outer casing 530 of the catheter unit 500 to a position as illustrated in FIG. 9A, thereby causing the first and second tubes 552, 554 to be drawn out through the end of the third tube 556, and injects liquid medicine. To stow the liquid medicine administering apparatus 400, as illustrated in FIG. 9B, the user slides the outer casing 530 with respect to the inner casing 510 so that the outer casing 530 is moved downward with respect to the inner casing, during which the stopper 512 is locked at the upper end of the long hole 538. As a result, the third tube 556 surrounds the first and second tubes 552, 554 as illustrated in FIG. 9B.

While the present invention has been particularly shown and described with reference to exemplary embodiments thereof, it will be understood by those skilled in the art that various changes in form and details may be made therein without depart ing from the spirit and scope of the invention as defined by th e appended claims.

### Industrial Applicability

The liquid medicine administering apparatus for endoscopic surgery, the liquid medicine controller and the liquid medicine administering apparatus having the same can be used for medical purpose.

## Claims

1. A liquid medicine administering apparatus for endoscopic surgery, comprising:
a head member comprising one or more liquid medicine inflow ports through which liquid medicine is introduced from a syringe, and one or more first gas inflow ports which are connected to the liquid medicine inflow ports and through which a gas from a gas supply line is introduced;
a tubular member connected to the head member and inserted into a human body, the tubular member comprising a liquid medicine passage through which the liquid medicine is discharged onto a surgical site; and
a valve unit installed on the head member to selectively connect the liquid medicine passage to the liquid medicine inflow ports or the first gas inflow ports, wherein
the liquid medicine introduced through the liquid medicine inflow ports is moved along the liquid medicine passage by an air provided from the gas inflow ports, to be discharged onto the surgical site.

2. The liquid medicine administering apparatus of claim 1, wherein the tubular member comprises:
an inner tube forming the liquid medicine passage;
an outer tube having a passage to receive the inner tube therein, wherein
the head member is slidably provided on the outer tube to cause an end of the inner tube to be selectively protruded outside an end of the outer tube.

3. The liquid medicine administering apparatus of claim 2, wherein the one or more liquid medicine inflow ports comprise a first liquid medicine inflow port connected to a first syringe filled with a first liquid medicine, and a second liquid medicine inflow port connected to a second syringe filled with a second liquid medicine, and
the one of more first gas inflow ports comprise a first gas inflow pipe connected to the first inflow port, and a second gas inflow pipe connected to the second inflow port.

4. The liquid medicine administering apparatus of claim 3, wherein the liquid medicine passage formed in the inner tube is partitioned into a first liquid medicine passage and a second liquid medicine passage through which the first and second liquid medicines are flowed, respectively, wherein
the first liquid medicine passage is connected to the first liquid medicine inflow port and the first gas inflow pipe, and the second liquid medicine passage is connected to the second liquid medicine inflow port and the second gas inflow pipe, and
the valve unit comprises,
a first valve body which selectively connects the first liquid medicine passage to the first liquid medicine inflow port or to the first gas inflow pipe,
a second valve body which selectively connects the second liquid medicine passage to the second liquid medicine inflow port or to the second gas inflow pipe, and
an operating lever which connects the first and second valve bodies for concurrent operating of the first and second valve bodies.

5. The liquid medicine administering apparatus of claim 3, wherein the first and second liquid medicines comprise a skin adhesive component.

6. The liquid medicine administering apparatus of claim 2, wherein the head member further comprises a second gas inflow port connected to the gas supply line, and
the tubular member has a passage formed between the inner tube and the outer tube to allow the air provided from the second gas inflow port to be discharged along with the liquid medicine at an end where the air and the liquid medicine are discharged.

7. A liquid medicine controller, comprising:
at least two liquid medicine inlets;
at least two liquid medicine outlets connected to the liquid medicine inlets; and
a valve member which connects the at least two liquid medicine inlets and the at least two liquid medicine outlets, or connects the at least two air inlets and the at least two liquid medicine outlets.

8. The liquid medicine controller of claim 7, wherein the valve member connects the liquid medicine inlets to the liquid medicine outlets and at the same time, blocks the air inlets from the liquid medicine outlets, or
connects the air inlets to the liquid medicine outlets and at the same time, blocks the liquid medicine inlets from the liquid medicine outlets.

9. The liquid medicine controller of claim 7, wherein the valve member is rotatably provided between the liquid medicine inlets and the liquid medicine discharge ports.

10. The liquid medicine controller of claim 7, wherein the valve member comprises a tubular valve body and a lever connected to the tubular valve body to be manually operated by a user, and the tubular valve body comprises a plurality of passing holes formed therein.

11. A liquid medicine administering apparatus for endoscopic surgery, comprising:
a syringe unit;
a liquid medicine controller; and
a catheter unit connected to the liquid medicine controller, wherein
the liquid medicine controller comprises,
at least two liquid medicine inlets;
at least two liquid medicine outlets connected to
the liquid medicine inlets; and
a valve member which connects the at least two liquid medicine inlets and the at least two liquid medicine outlets, or connects the at least two air inlets and the at least two liquid medicine outlets.

12. The liquid medicine administering apparatus of claim 11, wherein the catheter unit comprises:
a connecting member separably connected to the liquid medicine controller;
an inner casing connected to the connecting member;
a first and a second tubes provided in an interior space of the inner casing; and
an outer casing formed to surround at least part of the inner casing.

13. The liquid medicine administering apparatus of claim 12, wherein the outer casing is slidable in a lengthwise direction with respect to the inner casing.

14. The liquid medicine administering apparatus of claim 12, further comprising a third tube connected to the outer casing to surround the first and second tubes therein.
